# EUROPEAN PATENT SPECIFICATION

(11) **EP 0 613 358 B1**
(45) Date of publication and mention of the grant of the patent: **07.01.1999**
(21) Application number: 91920926.2
(22) Date of filing: 21.11.1991
(51) Int. Cl.: A61F 9/00, A61B 19/00

(54) **AN APPARATUS TO AUTOMATICALLY POSITION AND OPERATE SURGICAL INSTRUMENTS**
VORRICHTUNG ZUM AUTOMATISCHEN ANBRINGEN UND BETÄTIGEN CHIRURGISCHER INSTRUMENTE
APPAREIL DE POSITIONNEMENT ET DE MISE EN OEUVRE AUTOMATIQUES POUR INSTRUMENTS CHIRURGICAUX

(43) Date of publication of application: 07.09.1994
(73) Proprietor: PINTUCCI, Stefano, 00197 Roma (IT)
(72) Inventor: PINTUCCI, Stefano, 00197 Roma (IT)
(74) Representative: Iannone, Carlo Luigi
(86) International application number: IT9100099
(87) International publication number: WO9309738

(56) References cited:
- EP-A- 0 208 950
- EP-A- 0 239 409
- EP-A- 0 442 156
- DE-A- 3 433 581
- US-A- 2 480 737
- US-A- 4 173 980

## Description

The present invention relates to an apparatus to automatically position and operate surgical instruments, in particular for refractive keratic surgery, in particular lamellar refractive surgery.

More particularly, the invention relates to an apparatus of the said type, that allows surgical techniques to be carried out with an extreme safety and a high repeatability.

It is well-known that in all the fields of surgery one tries to realize equipments that allow to operate to a greater precision, and, moreover, allow surgeons having not a very long experience to carry out delicate operative surgeries.

This is even more true in those fields wherein it is operated upon delicate organs, and therefore is particularly felt in ophthalmological surgery.

In this field, a very important operation consists in lamellar refractive surgery (keratomyleusis).

Keratomyleusis is a surgical methodology well known to those skilled in the art, originally set by Prof. Barraquer.

It has developed during years through three techniques, and the relevant apparatuses have been realized by Barraquer himself, by Krumeich and Swinger and then by Ruiz.

Among the various equipments that make up the set of the surgical instruments necessary to operate, Barraquer's technique provides the use of a sucking metal ring which is applied on the eye of the patient and whereon the microkeratome slides.

In order to be able to achieve the exact keratic surface protrusion it is necessary to have a complete set of sucking metal rings at disposal.

Before carrying out the operation, one measures the intraocular pressure caused by the ring and measures, by means of a precalibrated lens, the lenticule diameter that would be obtained with that particular ring.

Only after verifying these variables and selecting the proper ring, one will be able to proceed to the section of the lamella.

Obviously, all these operations render the operation more difficult, less safe and longer.

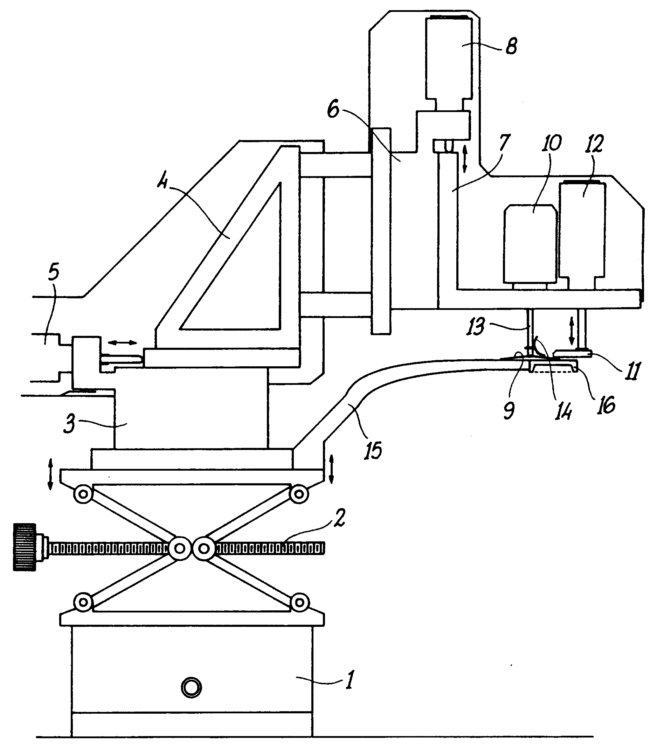

In EP-A-0 208 950 it is disclosed an apparatus to automatically position and operate surgical instruments for performing corneal transplants comprising:
- means for correctly positioning the equipment in correspondence with the operating table (2) (see figure 1);
- means for vertically raising all the apparatus (see page 13, second paragraph, first sentence, reference 7);
- a horizontal guide (9) (see page 13, second paragraph, second sentence);
- a first support formed by references (8) and (11), arranged slidably on the guide (9) (see page 13, second paragraph, second and third sentence);
- a horizontal linear actuator which determines the slide, in both direction, of the support member (8, 11) on the horizontal guide (see page 13, second paragraph and page 14, last paragraph, second sentence);
- a vertical guide (see figure 5), integral with the support member (8, 11), at right angle to the horizontal guide (considering that the rotating support 11 is turned such that instrument 18 of figure 5 is in the vertical position);
- an arm (66), integral with the first support member (8, 11) bearing at its free end a sucking ring (14) connected with a vacuum source (see page 17, first paragraph, first sentence);
- two members (58, 60) which are independently slidable in vertical direction (see references 50 and 59) with respect of the support member (11) by means of actuators (see page 25, third paragraph, last sentence), whereby member (60) carries a rod (61) to which a pad (62) is mounted, and member (58) carries the surgical instrument (52). The pad comprises a concave surface (77) (see page 18, first paragraph, sixth sentence), the periphery of which forms a plane. Thus, member (60) constitutes a second support member bearing the plane, whereby the member (58) supporting the surgical instrument is endowed with the second vertical actuator allowing raising and lowering of the instrument (52) in an autonomous manner with respect to the second support member (60), resulting in the possibility of having the plane and the instrument moved vertically together, as well as having a second, independent vertical movement of the instrument with respect of the plane.

In the techinque set by Krumeich and Swinger also the same type of sucking rings is employed.

In Ruiz's technique, on the contrary, a series is employed of pneumatic rings which present the same already mentioned problems.

Recently, in the Application for Italian Patent No. 48104-A/89 filed on June 21, 1989, an apparatus set has been suggested for carrying out myopical or hypermetropical keratomyleusis wherein a single sucking ring endowed with an adjustment ferrule is provided, for determining the diameter of the flap to be removed, and constituting a track for the sliding of the microkeratome and the precalibrated lens.

As is apparent, such a solution constitutes a considerable progress in comparison with the preceding apparatuses in that it allows to rapidly determine the diameter of the flap to be removed, to measure intraocular pressure and to carry out the refractive section.

Subsequently, it has been suggested, by the same Applicant, a further improvement of the latter apparatus, and particularly a sucking ring realized with an extremely simple mechanics, with a handle that allows a very safe positioning of the ring on the eye of the patient and able to effect decisely considerable excursions downwards and upwards.

All what has been disclosed above if on a hand has allowed the operation technique to be more and more improved, always maintains a very strict link between the result of the operation and the ability of the surgeon.

This is true in that the result is strictly connected with the ability of the surgeon.

Moreover, with these apparatuses of the manual type, it turns out to be very difficult to have a high repeatability of the operation.

In view of the above, the Applicant has thought of realizing an apparatus that allows the aforementioned operative technique to be executed in an automatic and absolutely repeatable manner.

An apparatus to automatically position and operate instruments for refractive keratic surgery, comprising:
a connector member for contact with an underlying support surface,
a raising member for lifting a surgical assembly vertically with respect to said base support member, said raising member being supported by said connector member, and said surgical assembly comprising:
   (a) a horizontal guide supported by said raising member,
   (b) a first support member slideably supported by said horizontal guide,
   (c) a horizontal linear actuator in contact with said first support member for sliding said first support member along said horizontal guide,
   (d) a vertical guide joined with said first support member,
   (e) a second support member in engagement with said vertical guide so as to be adjustable both in an up and a down vertical direction along a vertical axis, and said second support member including a horizontal platform,
   (f) a first vertical linear actuator in contact with said support member for adjusting said second support member and platform in both an up and a down vertical direction along the vertical axis,
   (g) an arm for operating and supporting a surgical instrument, said arm for operating and supporting a surgical instrument being supported by said horizontal platform,
   (h) a plane dimensioned for contact with an anatomical portion to be operated upon,
   (i) a second vertical actuator supported by said platform and being independently vertically adjustable with respect to said platform, said plane being joined with said second vertical actuator such that said plane is vertically adjustable in an autonomous manner with respect to said platform,
   (j) an arm having a first end connected to at least one of said raising member and said horizontal guide, and said arm having a free end extending below said member for operating and supporting a surgical instrument and said free end bearing a positioning member for positioning an anatomical portion to be operated upon.

Other feature are described in dependent claims 2-14.

The present invention viii be now disclosed, as a matter of illustration, but not of restriction, according to a preferred embodiment thereof represented in the figure of the annexed drawing wherein a schematic side view is shown of the apparatus according to the invention.

The apparatus shown in the figure provides a connector 1 of a magnetic type for the fastening to the operative cot (not shown), above which a member 2 is provided for raising all the apparatus on the cot, so as to be able to correctly position it.

The assembly connector 1-member 2 bears, integral with it, a horizontal guide 3 which serves to position, in a known manner, the whole proper surgical instrument with respect to the eye of a patient.

It bears, slideably, a support structure 4 wherewith the rest of the apparatus according to the invention is coupled in an integral manner, in the way that will be seen below.

Said support 4 is made to slide upon the guide 3 by the linear actuator 5, which preferably will be a continuous current motor, in that the type of action that it has to perform requires a continuous linear movement.

At the extremity opposite that for the coupling with the guide 3, the support 4 integrally bears a vertical guide 6 at right angles to the guide 3.

Upon said vertical guide 6 a second support 7 slides actuated by the linear actuator 8 which, in this case, will be preferably a stepper motor.

Said support 7 has a vertical arm, which slides upon the vertical guide 6 and a horizontal arm which bears the blade for cutting 9, and the relevant c.c. motor 10, and the plane 11 with the relevant vertical linear actuator 12.

On the arm 13 of the blade 9 a little spoon 14 for picking the cut lamella is provided.

Obviously, to the arm 13 a tool different from the blade 9 can be applied, e.g. a miller, to carry out further stages of the operative surgery.

To said member 2 an arm 15 is coupled in an integral, but removable manner, at its extremity that goes to find itself under the blade 9 and the plane 11, bearing a sucking ring 16.

The sucking system (not shown) of the sucking ring 16 can be provided inside the arm 15.

As will be seen in the following, the plane 11 allows the cut thickness to be adjusted, while the blade 13, raising and lowering itself integrally with the support 7, allows the diameter to be adjusted.

In the place of the arm 15 and of the ring 16 other members necessary to carry out subsequent stages of the operative surgery can be provided.

All the operation of the various actuators 5, 8, 10 and 12 can be controlled by a pre-programmed central unit instructable step by step during the operative surgery.

Compared with the sucking ring employed for the manual execution of keratomyleusis operation, the ring 16 hasn't to be endowed with the guides for the slide of the microkeratome, with the handle and with the raising and lovering mechanisms for finding the zero and determining the thickness of the cut or section.

To initiate an operative surgery with the apparatus represented in the figure, it is first of all necessary to proceed to set all the instruments to zero.

This is achieved by bringing, by means of the horizontal actuator 5 and the vertical actuator 8, the blade 9 into contact with the ring 16, after completely raising the plane 11.

The ring 16 will be electrically insulated with respect to the other components.

When the blade 9 and the ring 16 are in contact one is in the ideal position for the maximum diameter and maximum thickness section. This is the first reference point.

The actuator 12 also is insulated with respect to the actuator 10.

Then one lowers the plane 11 up to touch the sucking ring 16, i.e. in a potential condition of zero thickness section. This is the second reference point.

Then one reraises the whole by a known quantity, by means of the actuator 8.

At this point it is necessary to bring the eye of the patient in correspondence with the sucking ring 16.

The patient is layed down and is made to stare at a luminous point, marking the centre of the cornea.

After the anaesthesia and applying the usual operative techniques, one positions the apparatus on the operative cot, causing the sucking ring 16 to find itself above the eye of the patient.

Before applying the vacuum on the ring, one brings, through the actuator 5, all the instrumenrt in a withdrawn position, and makes the patient to stare upwards in such a manner as to make the centre of the sucking ring to correspond with the centre of the cornea.

Then one applies the vacuum and the eye is fixed in position.

The member 2 is no more touched. The system is stationary.

Then one measures the pressure of the eye and the proper operation can be initiated.

Through the actuator 5 the blade 9 and the plane 11 are brought onto the ring 16 and then, through the vertical actuator 8 one lowers the blade 9, kept firm, up to skim the eye of the patient.

The cornea of the patient being a conductor, when the blade 9 skims it one has another reference point of the system.

Indeed, from this point, the more the blade 9 is lowered, the greater the diameter of the section.

One withdraws the blade 9 backwards, horizontally, through the actuator 5 and lowers it by the quantity necessary to achieve the section of the desired diameter.

In this position, the plane 11 will go to find itself on the eye of the patient.

By acting now on the vertical actuator 12, by raising the plane 11, one determines the desired thickness of the section.

At this point, one actuates the motor 10, making the blade 9 to rotate and the horizontal actuator 5, thereby achieving the section.

After finishing the section, one raises the whole and brings back it.

To operate according to Ruiz's technique, it will suffice to execute the second section and then reapply the first removed lamella.

Where, on the contrary, it is desired to execute Krumeich's technique, it will suffice to remove the arm 15 with the sucking ring 16 and substitute it with an arm bearing a bench for working the lamella.

One executes the correction of the lamella which will thereafter be reapplied to the patient.

The blade 9, moreover, can be substituted by a miller for correcting the section where the existence of a step on the section is recognized.

The present invention has been described with specific reference to some its preferred embodiments, but it is to be understood that variations and/or modifications can be made by those skilled in the art, without so getting out of the relevant protection scope.

## Claims

1. An apparatus to automatically position and operate instruments for refractive keratic surgery, comprising:
a connector member (1) for contact with an underlying support surface,
a raising member (2) for lifting a surgical assembly vertically with respect to said base support member, said raising member being supported by said connector member, and said surgical assembly comprising:
(a) a horizontal guide (3) supported by said raising member (2),
(b) a first support member (4) slideably supported by said horizontal guide (3),
(c) a horizontal linear actuator (5) in contact with said first support member (4) for sliding said first support member (4) along said horizontal guide (3),
(d) a vertical guide (6) joined with said first support member (4),
(e) a second support member (7) in engagement with said vertical guide (6) so as to be adjustable both in an up and a down vertical direction along a vertical axis, and said second support member (7) including a horizontal platform,
(f) a first vertical linear actuator (8) in contact with said support member (7) for adjusting said second support member (7) and platform in both an up and a down vertical direction along the vertical axis,
(g) an arm (13) for operating and supporting a surgical instrument, said arm (13) for operating and supporting a surgical instrument being supported by said horizontal platform,
(h) a plane (11) dimensioned for contact with an anatomical portion to be operated upon,
(i) a second vertical actuator (12) supported by said platform and being independently vertically adjustable with respect to said platform, said plane (11) being joined with said second vertical actuator (12) such that said plane (11) is vertically adjustable in an autonomous manner with respect to said platform,
(j) an arm (15) having a first end connected to at least one of said raising member (2) and said horizontal guide (3), and said arm (15) having a free end extending below said member for operating and supporting a surgical instrument and said free end bearing a positioning member (16) for positioning an anatomical portion to be operated upon.

2. The apparatus according to claim 1, characterized in that it comprises a pre-programmed surgeon settable central unit for automatically controlling all the operation and the various functions of the apparatus.

3. The apparatus according to anyone of the preceding claims, characterised in that said horizontal linear actuator (8) is comprised of a continuous current motor.

4. The apparatus according to one of the preceding claims, characterized in that said first vertical linear actuator (12) is made up of a stepper motor.

5. The apparatus according to one of the preceding claims, characterized in that said second vertical linear actuator is made up of a stepper motor or a nonius.

6. The apparatus according to one of the preceding claims, characterized in that said member for operating and supporting the surgical instrument is comprised of a continuous current motor (10) and of a spindle (13) bearing, at its free end, in a fixed or removable manner, said surgical instrument.

7. The apparatus according to anyone of the preceding claims, characterized in that said surgical instrument is comprised of a rotating blade (9).

8. The apparatus according to anyone of claims 1 to 6, characterized in that said surgical instrument is comprised of an oscillating blade.

9. The apparatus according to claim 6, characterized in that a miller for correcting the section is mounted on said spindle.

10. The apparatus according to anyone of claims 6 to 9, characterized in that a spoon member (14) for taking in the cut lamella is provided on said spindle (13).

11. The apparatus according to anyone of the preceding claims, characterized in that said arm (15), is fixedly or removably connected with said positioning and raising means, bears, at its free end, in a position lower than the surgical instrument, a sucking ring (16) connected with the vacuum source.

12. The apparatus according to anyone of claims 1 to 10, characterized in that a bench for working the cut lamella is provided on said arm.

13. The apparatus according to anyone of the preceding claims, characterized in that said means for the positioning upon the cot are comprised of a magnetic system, of brackets or other similar mechanisms.

14. The apparatus according to anyone of the preceding claims, characterized in that said vertical raising means (2) are of a mechanical and/or pneumatic and/or hydraulic type.

## Patentansprüche

1. Vorrichtung zum automatischen Anbringen und Betätigen von chirurgischen Instrumenten für keratische Brechungschirurgie, enthaltend:
ein Zwischenglied (1) in Berührung mit einer Unterlagefläche;
ein Aufhebeglied (2) zum Aufheben einer chirugischen Anordnung senkrecht zum vorgenannten Trageglied, wobei das vorgenante Aufhebeglied durch das vorgenannte Zwischenglied abgestützt ist wobei vorgenannte chirugische Anordnung die folgenden Bestandteile enthält:
a) eine waagerechte, durch das vorgenannte Aufhebeglied unterstützte Führung (3);
b) ein erstes auf der vorgenannten waagerechten Führung (3) verschiebbar gelagertes Trageglied (4);
c) ein waagrechter linearer Antrieb (5) in Berührung mit dem vorgennnten ersten Trageglied (4) zur Verschiebung des vorgenannten ersten Trageglieds (4) längs der vorgenannten waagerechten Führung (3);
d) eine senkrechte, mit dem vorgenannten ersten Trageglied (4) verbundene Führung (6);
e) ein zweites Trageglied (7), das mit der vorgenannten senkrechten Führung (6) so in Eingriff steht, dass es sich auf- und abwärts längs einer senkrechten Achse verschieben kann;
f) ein erster senkrechter linearer Antrieb (8) in Berührung mit dem vorgenannten Trageglied (7) zur Verstellung des vorgenannten zweiten Tragegliedes (7) und einer Plattform auf- und abwärts längs der senkrechten Achse;
g) ein Arm zur Betätigung und Unterstützung eines chirurgischen Instrumentes, wobei der vorgenannte Arm (13) zur Betätigung und Unterstrützung eines chirurgischen Instrumentes auf der vorgenannten waagerechten Plattform gelagert ist;
h) eine flache, für die Berührung mit einem zu operienden anatomischen Körperteil bemessene Bahn (11);
i) ein zweiter, durch die vorgenannte Plattform getragener und gegenüber derselben senkrecht verstellbarer Antrieb (12), wobei diese Bahn (11) mit dem vorgenanten zweiten Antrieb (12) so verbuden ist, dass die vorgenannte Bahn (11) gegenüber der vorgenannten Plattform in senkrechter Richtung automatisch verstellbar ist.
j) ein Arm (15), dessen erstes Ende mindestens mit dem vorgenannten Aufhebeglied (2) oder mit der vorgenannten waagerechten Führung (3) verbunden ist, wobei der vorgenannte Arm (15) ein freies Ende das sich unterhalb des vorgenannten Gliedes zum Anbringen und Betätigen eines chirurgischen Instrumentes erstreckt und das vorgenannte freie Ende ein Stellglied trägt, das zur Anordnung eines zu operierenden anatomischen Teiles dient.

2. Vorrichtung nach Anspruch 1, dadurch gekennzeichnet, dass eine vorprogammierte, vom Chirurgen einstellbare Zentraleinheit vorgeschen ist, die zur automatischen Steuerung der ganzen Operation und der verschiedenen Funktionen der Vorrichtung dient.

3. Vorrichtung nach je einem der vorhergehenden Ansprüche, dadurch gekennzeichnet, dass der vorgenannte waagerechte linearer Antrieb (8) mit einem Gleichstrom-Motor versehen ist.

4. Vorrichtung nach je einem der vorhergehenden Ansprüche, dadurch gekennzeichnet, dass der vorgenannte senkrechte lineare Antrieb (12) aus einem Schrittmotor besteht.

5. Vorrichtung nach je einem der vorhergehenden Ansprüche, dadurch gekennzeichnet, dass der vorgenannte zweite lineare Antrieb aus einem Schrittmotor oder Nonius besteht.

6. Vorrichtung nach je einem der vorhergehenden Ansprüche, dadurch gekennzeichnet, dass das vorgenannte Glied zum Antrieben und Tragen des chirurgischen Instrumentes einen Gleichstrommotor (10) und eine Spindel enthält, die an ihrem freien Ende das vorgenannte chirurgische Instrument in abnehmbarer oder fester Weise trägt.

7. Vorrichtung nach je einem der vorhergehenden Ansprüche, dadurch gekennzeichnet, dass das vorgenannte chirurgische Instrument eine drehbare Lanzette (9) enthält.

8. Vorrichtung nach je einem der vorhergehenden Ansprüche 1 bis 6, dadurch gekennzeichnet, dass das vorgenannte chirugische Instrument eine Schwingklinge enthält.

9. Vorrichtung nach Anspruch 6, dadurch gekennzeichnet, das auf der vorgenannten Spindel ein Fräser zur Zurichtung des Schnittes angeordnet ist.

10. Vorrichtung nach je einem der Ansprüche 6 bis 9, dadurch gekennzeichnet, dass auf der vorgenannten Spindel (13) ein Löffelteil zur Aufnahme der geschnittenen Lamellen angeodnet ist.

11. Vorrichtung nach je einem der vorhergehenden Ansprüche, dadurch gekennzeichnet, dass der vorgenannte Arm (15) fest oder abnehmbar mit vorgenannten Stell- und Aufhebemitteln verbunden ist und an seinem freien Ende, in einer unterhalb des chirugischen Instrumentes liegenden Stelle, einen mit der Vakuumquelle verbundenen Saugring (16) trägt.

12. Vorrichtung nach je einem der vorhergehenden Ansprüche 1 bis 10, dadurch gekennzeichnet, dass auf dem vorgenannten Arm eine Fläche zur Bearbeitung der Lamelle vorgesehen ist.

13. Vorrichtung nach je einem der vorhergehenden Ansprüche, dadurch gekennzeichnet, dass die vorgenannten Mittel zur Aufstellung auf dem Operationssitz aus einem magnetischen System, aus Klauen oder aus anderen ähnlichen mechanischen Mitteln bestehen.

14. Vorrichtung nach je einem der vorhergehenden Ansprüche, dadurch gekennzeichnet, dass die vorgenannten Senkrechtaufhebemittel (2) mechanischer und/oder pneumatischer und/oder hydaulischer Art sind.

## Revendications

1. Appareil de positionement et de mise en ouvre automatiques pour la kèrato-chirurgie réfractive, comprenant:
- un élément de conneion (1) à contacter une surface de support au-dessous;
- un élément de levage (2) pour lever un ensemble chirurgique verticalement par rapport audit élément du support de base, ledit élément de levage étant supporté par ledit élément de connexion, et ledit ensemble chirugique comprenant:
- (a) un guide horizontal (3) supporté par ledit élément de levage (2);
- (b) un premier élément de suport (4) supporté à glissement par ledit guide horizontal (3);
- (c) un dispositif d'enttrainement linéaire horizontal (5) en contact avec ledit premier élément de support (4) pour faire glisser ledit premier élément de support le long dudit guide horizontal (3);
- (d) un guide vertical (6) joint audit premier élément de support (4);
- (e) un deuxième élément de support en engagement avec ledit guide vertical (6) de façon réglable verticalement en haut et en bas le long d'un axe vertical et ledit deuxième élément de support comprenant une plate-forme horizontale;
- (f) un premier dispositif d'entrainement linéaire vertical (8) en contact avec ledit élément de support (7) pour ajuster ledit deuxième élément de support (7) et la plate-forme verticalement en haut et bas le long de l' axe vertical;
- (g) un bras (13) pour actionner et supporter un instrument de chirurgie, ledit bras (13) pour actionner et supporter un instrument chirurgique étant supporté par ladite plate-forme horizontale;
- (h) un plan (11) dimensionné pour entrer en contat avec une partie anatomique à operée;
- (i) un deuxième dispositif d'entrainement vertical (12) supporté par ladite plate-forme et ajustable indépendamment en direction verticale par rapport à ladite plate-forme, ledit plan (11) étant joint audit deuxième dispositif d'entrainment vertical (12) de façon que ledit plan (11) est adjustable verticalement et indépendamment par rapport à ladite plate-forme;
- (j) un bras (15) ayant une premier extrémité joint au moins à un desdits élément de lavage (2) et guide horizontal (3) et ledit bras (15) ayant une extrémité libre s'étendant au-dessous dudit élément pour actionner et supporter un instrument de chirurgie et ladite extrémité libre portant un élément de positionnement (16) pour positionner une partie anatomique à operée.

2. Appareil selon la revendication 1, caractérisé en ce qui comprend une unité centrale pre-programmé, réglable par chirurgien, pour controler automatiquement tous les operations et les différentes fonctions de l'appareil.

3. Appareil selon l'une quelconque des revendications précédentes, caractérisé en ce que ledit dispositif d'entrainement linéaire horizontal (8) est pourvu d'un moteur à courant continu.

4. Appareil selon l'une quelconque des revendications précédentes, caractérisé en ce que ledit premier dispositif d'entrainement linéaire vertical est un moteur pas à pas.

5. Appareil selon l'une quelconque des revendications précéndetes, caractérisé en ce que ledit deuxième dispositif d'entrainement linéaire vertical est un moteur pas à pas ou un vernier.

6. Appareil selon l'une quelconque des revendications précéndetes, caractérisé en ce que ledit élément pour actionner et supporter l'instrument de chirurgie consiste d'un moteur à courant continu (10) et d'un axe (13) portant, à sa extrémité libre, de façon fix ou amovible, ledit instrument chirugique.

7. Appareil selon l'une quelconque des revendications précédentes, caractérisé en ce que ledit instrument chirugique est une lame rotative.

8. Appareil selon l'une quelconque des revendications précédentes, caractérisé en ce que ledit instrument chirurgique est une lame oscillante.

9. Appareil selon la revendication 6, caractérisé en ce que une fraiseuse est montée sur ledit axe.

10. Appareil selon l'une quelconque des revendications 6 à 9, caractérisé en ce que sur ledit axe (13) est placé un élément à cuiller pour prélever les lamelles coupées.

11. Appareil selon l'une quelconque des revendications précédentes, caractérisé en ce que ledit bras (15) est joint en façon fix ou amovible avec lesdits moyens de positionnement et lavage et porte à sa extrémité libre, dans une position plus bas par rapport audit instrument de chirurgie, une bague d'aspiration (16) jointe à une source de vide.

12. Appareil selon l'une quelconque des revendications précédentes 1 à 10, caractérisé en ce que sur ledit bras un banc est prévu pour élaborer les lamelles coupées.

13. Appareil selon l'une quelconque des revendications précédentes, caractérisé en ce que lesdits moyens pour le positionnement sur le lieu d'operation sont pourvus d'un système magnetique, des ètagères ou des mécanismes similaires.

14. Appareil selon l'une quelconque des revendications précédentes, caractérisé en ce que lesdits moyens de lavage verticale (2) sont d'un type mécanique, et/ou pneumatique et/ou hydraulique.
